(19) Europäisches Patentamt

European Patent Office

Office européen des brevets

(11) **EP 1 623 668 A1**

(12) **EUROPEAN PATENT APPLICATION**

(43) Date of publication:
08.02.2006 Bulletin 2006/06

(51) Int Cl.:
*A61B 5/026* (2006.01)

(21) Application number: 05107015.9

(22) Date of filing: 29.07.2005

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 02.08.2004 JP 2004225665
18.03.2005 JP 2005080340

(71) Applicant: Nihon Seimitsu Sokki Co., Ltd.
Gunma 377-0293 (JP)

(72) Inventor: Yamakoshi, Yoshiki
371-0836, Gunma (JP)

(74) Representative: Betten & Resch
Patentanwälte,
Theatinerstrasse 8
80333 München (DE)

(54) **Evaluation of blood fluidity**

(57) To easily evaluate a fluidity of blood with high sensitivity. By winding a pressure bag body (10) around the surface of a skin of a finger tip (100) or the like of an examinee, and applying a compression thereto, the blood in a capillary blood vessel under the skin is flown out to the surroundings, and by irradiating the skin where the oppression is applied with light from a light emitting element (21), and measuring a degree of the light absorption as a light reception quantity of a light receiving element (22), thereby optically measuring the change of a quantity of blood in the capillary blood vessel, and obtaining the quantity of blood in the capillary blood vessel with passage of time after applying the oppression, the fluidity of the blood (mainly viscosity μ) is evaluated.

FIG. 1

EP 1 623 668 A1

**Description**

TECHNICAL FIELD OF THE INVENTION

The present invention relates to a method and an apparatus capable of easily evaluating a fluidity of blood (mainly viscosity of blood) from outside.

BACKGROUND ART

For example, in recent years, there has been a strong demand for objectively recognizing a viscosity of blood for a health maintenance, which is not easily executed, because in order to recognize the viscosity of the blood, generally a blood collection and an inspection are required.

Conventionally, although not to recognize the viscosity of the blood, an attempt has been made to evaluate a cardiac function and a blood pressure, and the degree of sclerosis of an artery, by objectively measuring a peripheral circulation state of a living body. The apparatus of this kind comprises a pressurizing device for pressuring a prescribed part of the living body to cause a congestion of blood at this part; a blood recovery quantity detecting device for optically detecting a recovery blood quantity to the prescribed part of the living body when the oppression of the pressurizing device is canceled; and a blood recovery quantity display device for displaying the change in the recovery blood quantity detected by the blood recovery quantity detecting device on a graph with passage of time along a prescribed time axis, to thereby evaluate a function by a returning speed of the blood when a condition is restored from a blood congestion state. (Patent document 1) Japanese Patent Laid Open No.11-89808

However, in a technique disclosed in the patent document 1 in which changes of blood flow is measured when the oppression is released, all blood systems are concurrently filled with blood when the oppression is released. Therefore, even if a wavelength of light and an interval of optical elements are optimized, it is difficult to completely differentiate an arterial system and a capillary vascular system. Also, under the influence of the pressure of the blood sent out from a heart, specifically under the influence of a blood pressure, a correction is difficult to be made, because of an ever-changing blood pressure. Further, the blood pressure pulsates in accordance with a pulse of the heart, and therefore a pulsation component must be removed. However, it is difficult to remove the pulsation component. Accordingly, although blood flow data of only the capillary vascular system is required for evaluating the viscosity of the blood, a viscosity evaluation with high sensitivity is difficult, because unnecessary information such as an artery is included in the data thus measured.

SUMMARY OF THE INVENTION

In view of the above-described circumstances, an object of the present invention is to provide a method and an apparatus capable of easily evaluating a fluidity of blood with high sensitivity.

Therefore, the present invention takes several aspects as follows.

In a first aspect, a method of evaluating a fluidity of blood is provided, comprising: applying an oppression on a surface of a skin of an examinee; measuring the change of a remaining blood quantity under the skin with passage of time caused by a blood outflow from the capillary blood vessel under the skin; and evaluating the resistance to a blood flow by its measurement data.

In a second aspect, a method of evaluating a fluidity of blood is provided, comprising: making blood in a capillary blood vessel under the skin flow out to the surroundings by applying an oppression on a surface of a skin of a predetermined portion of an examinee; optically measuring the change of a blood quantity in the capillary blood vessel by measuring the degree of light absorption when the surface of a skin of a predetermined portion of an examinee is irradiated with light; and evaluating the fluidity of the blood by obtaining the change of the blood quantity in the capillary blood vessel with passage of time after the oppression is applied thereto.

In a third aspect, the method of evaluating the fluidity of blood according to the second aspect is provided, wherein as an optical detector for measuring the degree of the light absorption when the skin of the predetermined portion is irradiated with light, combination of a light emitting element which irradiates the surface of the skin with light and a light receiving element which receives a returning light from under the skin is used, to set relative positions between the light emitting element and the light receiving element and the wavelength of an irradiating light, so that although the light reaches the capillary blood vessel under the skin, it does not reach a blood vessel such as an arteriole present in a location further deeper than the capillary blood vessel.

In a fourth aspect, the method of evaluating the fluidity of blood according to the third aspect is provided, comprising:

arranging the light emitting elements at a plurality of regular intervals and emitting the light from each light emitting element while shifting the time, collecting a plurality of measurement data through the light receiving element, and

evaluating a fluidity of the blood based on the data obtained by averaging the plurality of measurement data.

In a fifth aspect, the method of evaluating the fluidity of blood according to any one of the second to fourth aspects is provided, comprising: calculating a blood viscosity evaluation parameter from the data of the change of the blood quantity with passage of time; and based on the parameter thus obtained, evaluating the viscosity of the blood.

In a sixth aspect, the method of evaluating the fluidity of blood according to the third aspect is provided, comprising:

calculating a hemoglobin concentration (which is a ratio of a quantity of hemoglobin when the whole tissue is defined as 1, and the values are varied from 0 to 1) in the tissue of a predetermined portion where the oppression is applied based on a light reception intensity, and evaluating the fluidity of the blood based on the change of the hemoglobin concentration with passage of time.

In a seventh aspect, the method of evaluating the fluidity of blood according to the sixth aspect is provided, comprising: evaluating the viscosity of the blood, which is one element of the fluidity of the blood, based on a time wherein the hemoglobin concentration is decreased from a first predetermined value which is already reduced and sufficiently smaller than the value before applying the oppression, to a further smaller second predetermined value.

In an eighth aspect, the method of evaluating the fluidity of blood according to the seventh aspect is provided, wherein the second predetermined value is about half of the first predetermined value.

In a ninth aspect, the method of evaluating the fluidity of blood according to any one of the sixth to eight aspects is provided, wherein a blood viscosity evaluation parameter is calculated from the data of a light reception intensity of the light receiving element, and the relation between the blood viscosity evaluation parameter and the hemoglobin concentration is shown in a graph.

In a tenth aspect, an apparatus for evaluating a fluidity of blood is provided, comprising: a pressurizer for applying oppression on the surface of a skin of an examinee; an optical detector for optically measuring a degree of a light absorption by irradiating the skin of a part where the oppression is applied with light; a calculator for calculating the change of a blood quantity in a capillary blood vessel under the skin with passage of time by measurement data of the optical detector after applying pressure; and a display device for displaying an evaluation content of the fluidity of the blood based on a calculation result by the calculator.

In an eleventh aspect, the apparatus for evaluating the fluidity of blood according to the tenth aspect is provided, comprising:

the pressurizer having a pressure bag body for applying an oppression force to the skin by introducing a pressure air; and

the optical detector having the combination of a light emitting element for irradiating the surface of a skin with light and a light receiving element for receiving a returning light from under the skin, wherein the light emitting element and the light receiving element are provided in a part touching on the skin by the pressure bag body or the part pushed against the skin by an expansion of the pressure bag body.

In a twelfth aspect, the apparatus for evaluating the fluidity of blood according to the eleventh aspect is provided, wherein relative positions between the light emitting element and the light receiving element and a wavelength of an irradiating light are set so that although the light reaches a capillary blood vessel under the skin, it does not reach a blood vessel such as an arteriole present in a location further deeper than the capillary blood vessel.

According to the first aspect and the second aspect, the resistance to the blood flow is evaluated based on a speed of a blood outflow from the capillary blood vessel when the oppression is applied on the surface of the skin. Therefore, the fluidity of the blood (mainly the viscosity of the blood) can be evaluated with high sensitivity, without being influenced by the blood flow such as an artery and a vein present in a location further deeper than the capillary blood vessel. Specifically, the artery and the vein originally have a lower vascular resistance compared with that of the capillary blood vessel, and the blood flows out at high speed when the oppression is applied. Therefore, only the outflow of the blood remaining in the capillary vascular system can be measured, under no influence of the artery and the vein, and based on a measurement result, the fluidity (mainly viscosity) of the blood can be evaluated with high sensitivity. Particularly, in the second aspect, the change of the blood quantity is optically measured from the outside by using the light absorption by hemoglobin. Therefore, the structure for measurement can be simplified.

According to the third aspect, as the means for optical measurement, the combination of the light emitting element and the light receiving element is used, and the relative position between the light emitting element and the light receiving element and the wavelength of the irradiating light are set, so that the light reaches only the capillary blood vessel under the skin, and therefore the influence of the blood flow such as the artery present in the location deeper than the capillary blood vessel under the skin can be eliminated as much as possible, and the fluidity of the blood can be evaluated with high sensitivity.

According to the forth aspect, the fluidity of the blood is evaluated based on an average value of the plurality of measurement data obtained by using a plurality of light emitting elements. Therefore, it is possible to reduce the influence of the difference in the density of capillary blood vessels between measurement parts.

According to the fifth aspect, the blood viscosity evaluation parameter is calculated by the data of the change of the blood quantity with passage of time, and based on the parameter thus obtained, the viscosity of the blood is evaluated. Therefore, even when an autonomous vascular constriction of an artery system occurs, without depending on this situation, the viscosity of the blood can be evaluated with high precision. In addition, for example, by observing the change of the parameter per every unit time, information on the deformation of a blood cell or the like included in the blood can be grasped.

According to the sixth aspect, the hemoglobin concentration in the tissue of the living body is calculated based on the light reception intensity of the light receiving element, and the fluidity of the blood is evaluated based on the change of the hemoglobin concentration with passage of time. Therefore, it is absolutely possible to evaluate the viscosity of the blood based on the hemoglobin concentration.

For example, the hemoglobin concentration in the tissue of the living body is changed in association with the passage of time after applying the oppression (is changed in association with the blood outflow). However, when the viscosity of the blood is evaluated based on the change of the hemoglobin at a large concentration stage, it is possible to know viscosity characteristics of the whole blood as a multi-phase flow especially composed of a red blood cell and a blood plasma (which relates to a so-called coefficient of kinematic viscosity, probably corresponding to a level of sticky condition of the flow of the blood). Also, when the viscosity of the blood is evaluated based on the change of the hemoglobin concentration at a sufficiently small concentration stage, it is possible to know the viscosity characteristic that reflects the change of a deformation efficiency of the red blood cell caused by a sufficiently small diameter of the capillary blood vessel.

Moreover, "the concentration serves as a parameter for determining a good/bad blood flow to the capillary blood vessel" can be appropriately said for the hemoglobin concentration in the tissue of the living body. Therefore, by the measurement (or estimation) of the hemoglobin concentration, usability can be exhibited for the evaluation of a risk factor of bedsore (pressure sore) caused by a circulatory disorder to the capillary vascular system, a prevention of frostbite (including a mild symptom such as chilblain), and the evaluation of a skin regenerating property after external injury.

According to the seventh and eighth aspects, the viscosity of the blood, which is one element of the fluidity of the blood, is evaluated, based on the time required for reducing the hemoglobin concentration from a first predetermined value which is already reduced and is sufficiently smaller value than that before applying the oppression, to the second predetermined value which is further smaller than the first predetermined value. Therefore, the viscosity of the blood can be evaluated at the stage where the blood is sufficiently flown out from the capillary blood vessel, namely, at the stage free of the influence of artery-vein shunt. Particularly, in the eighth aspect, the time required for reducing the hemoglobin concentration by half is measured, and based on the time thus obtained, the viscosity is evaluated. Therefore, the viscosity can be evaluated in a short period of time.

According to the ninth aspect, the relation between the blood viscosity evaluation parameter and the hemoglobin concentration is shown by the graph, with the hemoglobin concentration taken on the abscissa axis, and the blood viscosity evaluation parameter taken on the ordinate axis. Therefore, only a required level free of the artery-vein shunt can be shown. Also, according to this graph, only the relation between the blood viscosity evaluation parameter and the hemoglobin concentration is shown. Therefore, there is neither dependency on an initial blood quantity, nor dependency on an elapsed time after applying oppression. Further, according to this graph, rheological properties of the blood (condition of changing the viscosity according to the diameter of the blood vessel, (corresponding to the quantity of the hemoglobin, here)) can be confirmed at a glance.

According to the tenth aspect, the fluidity of the blood is evaluated based on the speed of the blood outflow from the capillary blood vessel when the oppression is applied on the surface of the skin by the pressurizer, and the evaluation content is displayed on a display portion. Therefore, differently from the measurement when the pressure is released, the fluidity of the blood (mainly the viscosity of the blood) can be evaluated with high sensitivity under no influence of the blood flow of the artery and the vein. Specifically, the artery and the vein are originally have a lower vascular resistance than that of the capillary blood vessel, and the blood flows out at a high speed when the oppression is applied thereto. Therefore, only the outflow of the blood remained in the capillary vascular system can be measured under no influence of the artery and the vein, and based on the measurement result, the fluidity (mainly viscosity) of the blood can be evaluated with high precision.

Note that the pressure applied on the skin may be determined to be large enough to make the blood totally flows out from the capillary blood vessel. Therefore, the apparatus can be simply formed in a pen-type shape, in which an LED and a photo transistor are attached on the tip end thereof, so as to be pushed into the skin.

According to the eleventh aspect, the light emitting element and the light receiving element are provided in the part where the pressure bag body touches on the skin or the part pushed against the skin by the expansion of the pressure bag body. Therefore, the outflow of the blood from inside of the capillary blood vessel can be optically measured with high sensitivity. Also, according to the twelfth aspect, the influence of the blood flow such as the artery present in the location deeper than the capillary blood vessel under the skin can be eliminated as much as possible. Therefore, the fluidity of the blood can be evaluated with high sensitivity.

BRIEF DESCRIPTION OF THE DRAWINGS

Fig.1 is a block diagram of an apparatus for measuring a viscosity of blood (apparatus for evaluating the fluidity of the blood) of an embodiment of the present invention.
Fig.2A is a sectional view of a pressure bag body (cuff) in the same apparatus, and Fig.2B is an explanatory view of an essential part of another example.
Fig.3A is a characteristic view showing the change of a pressure introduced to the pressure bag body, and Fig.3B is a characteristic view showing the change of a light reception quantity of a light receiving element.
Fig.4 is a view showing a specific measurement data of light reception quantity in case of a low blood viscosity.
Fig.5 is a view showing the specific measurement data of light reception quantity in case of a high blood viscosity.
Fig.6 is a view showing an electric circuit model of an artery.
Fig.7 is a view showing the electric circuit model of a vein.
Fig.8 is a view showing the electric circuit model of a capillary blood vessel.
Fig.9 is a view showing the electric circuit model of the capillary blood vessel when applying pressure.
Fig. 10 is a schematic view showing kinetics of a blood vessel when applying pressure from outside.
Fig.11 is a view used for explaining the meaning of an evaluation parameter.
Fig.12 is a view used for explaining the meaning of the evaluation parameter.
Fig.13 is an explanatory view for the light reception intensity when light propagates through a plurality of layers with different hemoglobin concentration.
Fig.14 is a view showing the condition of the change of the hemoglobin concentration after applying pressure.
Fig.15 is a view showing a flow of processing.
Fig.16 is a view for physically explaining the meaning of $t_2 - t_1$.
Fig.17 is a view showing an actual measurement data.
Fig. 18 is a view showing a time change of the hemoglobin concentration.
Fig.19 is a view showing the actual measurement data.
Fig.20 is a view showing the actual measurement data.
Fig.21 is a view showing the actual measurement data.
Fig.22 is a view showing the actual measurement data.
Fig.23 is a view showing the actual measurement data.

BEST MODE FOR CARRYING OUT THE INVENTION

Best modes for carrying out the present invention will be explained with reference to the drawings hereafter.

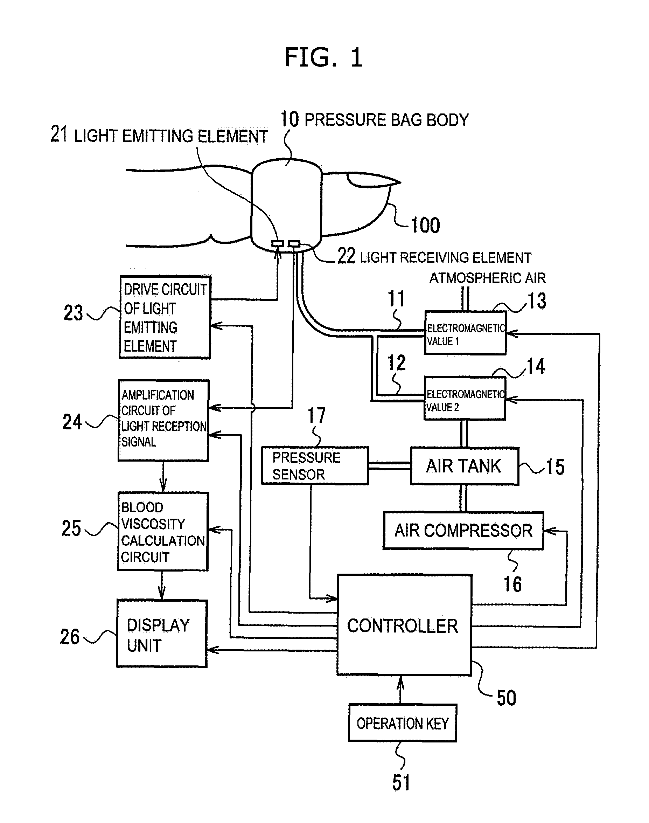

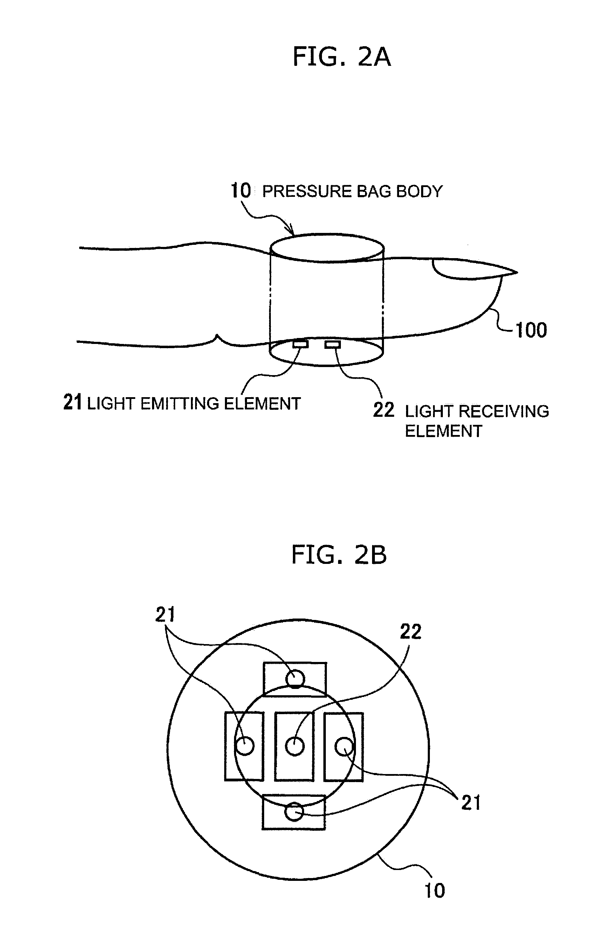

Fig. 1 is a block diagram showing a schematic constitution of an apparatus for measuring a blood viscosity (Apparatus for evaluating fluidity of blood), Fig.2A is a sectional view of a pressure bag body (caff) mounted on a finger, and Fig.2B is an explanatory view of an essential part of another example (as will be described later).
The blood viscosity measurement apparatus comprises: a pressurizer based on a pressure bag body (cuff) 10 mounted on a finger tip 100 of an examinee, for applying oppression to the surface of a skin of an abdomen side of the finger tip 100; a combination of a light emitting element 21 and a light receiving element 22 as an optical detector for optically measuring a degree of light absorption by irradiating a skin of a portion where the oppression is applied with light; a blood viscosity calculation circuit (calculator) 25 for calculating a blood quantity in a capillary blood vessel under the skin with passage of time by measurement data of the light receiving element 22 from the time point where the oppression is applied; and a display unit (display device) for displaying an evaluation content of the fluidity of the blood based on a calculation result of the blood viscosity calculation circuit 25.
The light emitting element 21 is driven and controlled by a drive circuit 23 for light emitting element, and a signal of the light receiving element 22 is amplified by an amplification circuit 24 for a light reception signal, and then inputted in the blood viscosity calculation circuit 25. Note that the number of the light emitting elements 21 and the light receiving elements 22 are not particularly limited. For example, a plurality of light receiving elements 22 are provided, and a plurality of light reception signals from each light receiving element 22 are averaged, and an evaluating calculation of the blood viscosity may be thereby performed. Then, the influence of error caused by the location of the oppression can be alleviated.

The pressure bag body 10 applies oppression force to the skin by introducing a pressure air, and connected to an atmosphere through an air introducing pipe 11 and a first electromagnetic valve 13, and also connected to an air tank 15 through the air introducing pipe 12 and a second electromagnetic valve 14. A pressure air is introduced into the air tank 15 from an air compressor 16, and the pressure in the air tank 15 can be controlled in a predetermined pressure, by driving and controlling the air compressor 16 by a controller 50, in accordance with a signal of a pressure sensor 17 for detecting the pressure of the air tank 15. Also, the controller 50 controls the supply and discharge of the pressure air to the pressure bag body 10, by controlling the first electromagnetic valve 13 and the second electromagnetic valve 14. Here, devices from the air compressor 16 to the pressure bag body 10 correspond to the pressurizer. Note that the controller 50 drives and controls all of the drive circuit 23 for light emitting element, the amplification circuit 24 for light reception signal, the calculation circuit 25 for blood viscosity, and the display unit 26. In addition, an operation signal is inputted in the controller 50 from an operation key 51.

The light emitting element 21 as the optical detector irradiates the surface of the skin with light, and the light receiving element 22 receives the returning light from under the skin. As shown in Fig.2A, the light emitting element 21 and the light receiving element 22 are arranged in a part of the pressure bag body 10 touching on the skin, so as not to be affected by external light as much as possible.

Next, a principle of the viscosity measurement of blood will be explained.

The capillary blood vessel is present in a shallow location under the skin, and the artery and the vein are present in a deeper location. When the oppression is applied to the skin, the blood flows out to other part at a high speed, because the artery and the vein have greater passage diameters. However, the blood in the capillary blood vessel flows out to the surroundings at a low speed, because the capillary blood vessel has a smaller passage diameter and a greater passage resistance. When the capillary blood vessel is pressured, a blood vessel passage is further narrowed, and therefore an outflow quantity is further reduced, under the influence of the change of the viscosity such as an aggregation of red blood cell and deterioration in deformation efficiency.

The time change (speed) of the blood outflow is dependent on a vascular resistance (the viscosity of the blood is given as one element thereof). Therefore, in this embodiment, by optically detecting the time change of the blood outflow from the capillary blood vessel, the viscosity, as one element of the vascular resistance, is estimated.

Generally, the hemoglobin and other living body constituting materials in a living body largely absorbs a visible light, and the visible light hardly transmits the living body. However, when the blood flows out from the capillary blood vessel when the oppression is applied to the skin, the blood (hemoglobin) is reduced along with the blood outflow, and light absorption is reduced in the living body compared to a case before applying the oppression. Specifically, the blood in the capillary blood vessel flows out by applying the oppression, and the light transmits the living body in association with a reduction in the blood quantity (quantity of hemoglobin).

Further, the light with short wavelength hardly transmits the living body. Specifically, it appears that to observe the light with short wavelength, with which the living body is irradiated, is to observe the light propagated through the capillary blood vessel of directly under the skin. By using this principle, the condition of the blood outflow in the capillary blood vessel due to the oppression applied thereto can be observed, thus making it possible to estimate the change of the hemoglobin concentration in the tissue of the living body.

In this case, preferably, the light emitting element 21 and the light receiving element 22 are set so as to detect only the blood quantity of the capillary blood vessel under the skin, and so as not to detect the blood quantity of the artery and the arteriole. Therefore, relative positions of the light emitting element 21 and the light receiving element 22 are made close to each other, so that the blood flow of a shallow part can be detected with high sensitivity. In addition, the wavelength of an irradiating light is not set in red or infrared region, but set in blue or a short wavelength region close to the blue.

This is because the light with longer wavelength hardly scatters in the midpoint, and the light invades into a deep portion (reach the artery), while the light with shorter wavelength does not reach the deep portion (does not reach the artery), but returns from a shallow position. Specifically, the light emitted from the light emitting element 21 invades into the skin, repeats scattering inside, and a part of the light returns to the surface of the skin. However, there is a blood vessel in the passage of the light, and the returning light is absorbed in the blood. Therefore, by using the light with short wavelength, without being affected by the light absorption by the blood in the arterioles and arteries of the deep portion under the skin, the light is absorbed only in the blood in the capillary blood vessel of the shallow portion, so that the light returns to the surface of the skin.

For example, when a blue LED is used as the light emitting element 21, the photo transistor is arranged as the light receiving element 22 within 3 mm distance from the position of the blue LED.

Next, a measurement method will be explained.

The pressure bag body 10 is wound around the finger tip for measurement. At this time, the first electromagnetic valve 13 is opened and an inner pressure of the pressure bag body 10 is set at the same pressure as the atmospheric pressure. In addition, the electromagnetic valve 2 is closed. In response to power-on of the apparatus, air is fed into the air tank 15 from the air compressor 16. The pressure in the air tank 15 is monitored by the pressure sensor 17, and when the pressure in the air tank 15 reaches a predetermined value, the air compressor 16 stops feeding of the air.

In this state, when a measurement start instruction is emitted from the operation key 51, the first electromagnetic valve 13 is closed, and the second electromagnetic valve 14 is opened. Then, the pressure air in the air tank 15 is introduced into the pressure bag body 10, thereby setting at almost the same air pressure as that in the air tank 15, to apply oppression to the skin of the finger. The light emitting element 21 and the light receiving element 22 are provided in a surface of the pressure bag body 10 touching on the finger, and optical measurement is performed immediately after start. Then, the signal from the light receiving element 22 to the time passage is amplified and inputted in the calculation circuit 25 for blood viscosity, and data showing a performance of the blood (vascular resistance) flowing through the capillary blood vessel is calculated from a time change ratio of the signal of the light reception quantity (light reception intensity). The data includes an index showing a blood fluidity and a blood viscosity in the capillary blood vessel, which is displayed by the display unit 26.

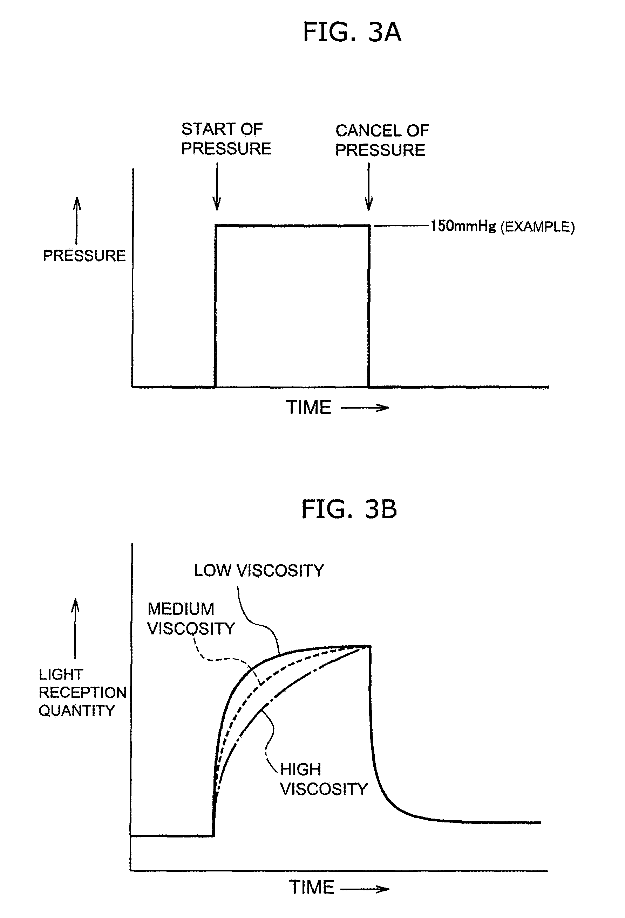

Fig. 3A shows the change of the pressure to be introduced to the pressure bag body 10, and Fig.3B shows the change of the light reception quantity of the light receiving element 22. When the oppression force is applied to the surface of the skin, the light reception quantity of the light receiving element 22 is changed, in association with the outflow of the blood of the capillary blood vessel under the skin to the surroundings. Then, the degree of the level of the viscosity of the blood can be estimated, in accordance with a difference in a manner for changing the gradient of a shoulder portion of a time change wave.

The flow is given as an example in principle as follows:

- large volume of blood quantity → greater light absorption → small volume of light reception quantity
- small volume of blood quantity → small light absorption → large volume of light reception quantity

Therefore, the principle leads to the next flow and evaluation is made as follows:

- large volume of light reception quantity - small light absorption → small volume of blood quantity → blood escapes easily by oppression (low viscosity)
- small volume of light reception quantity - greater light absorption → large volume of blood quantity → blood hardly escapes by oppression (high viscosity)

In this case, the vascular resistance is evaluated based on the time change of the blood outflow from the capillary blood vessel when the oppression is applied to the surface of the skin. Therefore, the fluidity of the blood (mainly viscosity of the blood) can be evaluated with high sensitivity, without being affected by the blood flow such as the artery and the vein present in the location further deeper than the capillary blood vessel. Specifically, the artery and the vein have a lower vascular resistance than that of the capillary blood vessel, and when the oppression is applied thereto, the blood flows out at a high speed. Therefore, under no influence of the artery and the vein, only outflow of the blood remaining in the capillary vascular system can be measured, and based on the measurement result, the fluidity (mainly viscosity) of the blood can be evaluated with excellent precision. Particularly, the change of the remaining blood quantity under the skin is optically measured from the outside, by using light absorption properties of the hemoglobin, and therefore a measurement structure can be simplified.

The skin is preferably pressed with strength greater than a maximum blood pressure in vivo. This is because the blood in the blood vessel of the pressure portion is required to be discharged to the surroundings by partially stopping a blood circulation naturally performed in the body. However, there is no thick artery in the finger tip and an ear lobule (capable of measuring not with the finger tip but with the ear lobule), there is only the arteriole as the artery, and the blood pressure in the arteriole is lower than that of the artery. Therefore, the oppression to be applied may not necessarily be higher than an estimated maximum blood pressure, thus relieving the load of the examinee.

In addition, the observation time of the blood flow after applying the oppression may be within approximately several seconds after applying the oppression. This is because the data obtained during this time reflects well the change of the blood fluidity. In the above example, although two electromagnetic valves 13 and 14 are used, they can be replaced with one three-way valve.

Actually, a measurement error is possibly generated, by different density of the capillary blood vessels depending on the examinee, and the different density of the blood vessels depending on a measurement part. Therefore, in order to reduce the measurement error, there is a method in which a plurality of light emitting elements are arranged at almost regular intervals around one light receiving element, and the lighting of the light emitting elements is performed at a time shifted in order, and the measurement in different areas of the skin is simultaneously performed and averaged.

Specifically, as shown in Fig.2B, a plurality of light emitting elements 21 are arranged at regular intervals, and by casting the light from each light emitting element 21, a plurality of measurement data are collected through the light receiving element 22. Then, based on the data obtained by averaging the plurality of measurement data, the fluidity of the blood is evaluated. In this way, the influence of the difference in the density of the capillary blood vessel between measurement parts can be reduced, and the evaluation with small error can be performed.

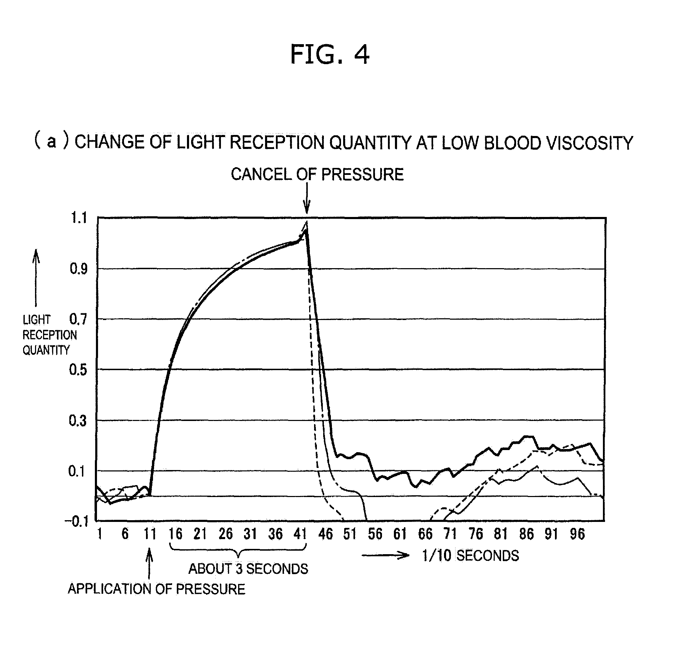

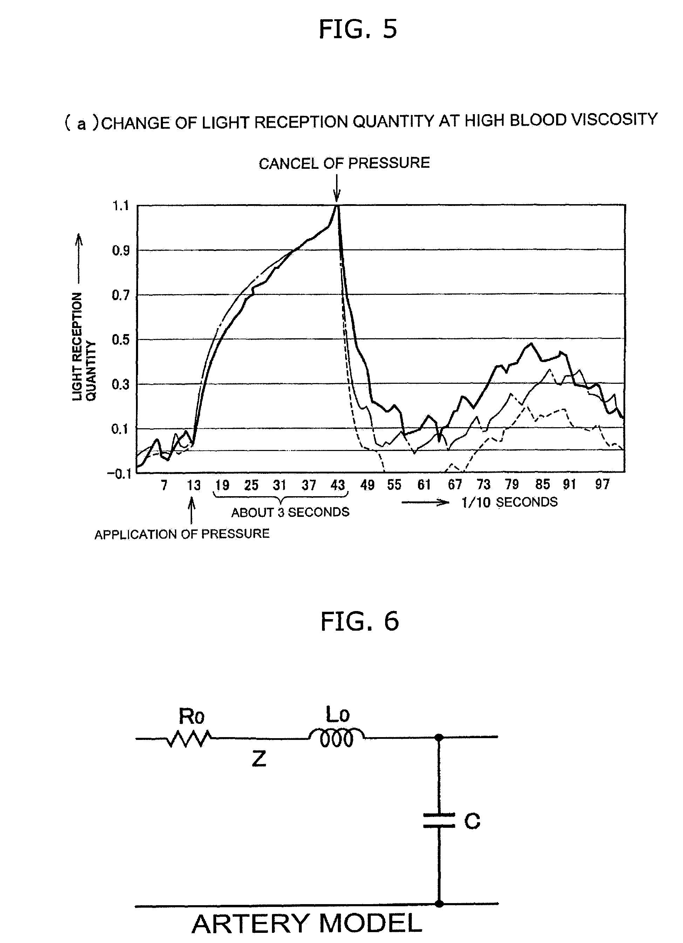

Fig.4 and Fig.5 show the measurement data actually collected. Fig.4A shows the change of the light reception quantity, and Fig.4B shows the change of the blood viscosity evaluation parameter calculated based on the change of the light reception quantity thus obtained.

In the measurement data of Fig. 4, in Fig. 4A, the gradient of a rising shoulder portion of a wave of the light reception quantity is loose, and in Fig.4B, the numeric value of a parameter a corresponding thereto is large. Meanwhile, in the measurement data of Fig. 5, in Fig. 5A, the gradient of the rising shoulder portion of the wave of the light reception quantity is steep, and in Fig.5B, the numeric value of the parameter corresponding thereto is small. Therefore, when both examples of Fig.4 and Fig.5 are compared, the blood viscosity is evaluated to be low in Fig.4, and the blood viscosity is evaluated to be high in Fig.5.

As shown in the examples of Fig.4 and Fig.5, when the blood viscosity evaluation parameter a is calculated from the measurement data of the light reception quantity corresponding to the blood quantity under the skin, the viscosity of the blood can be objectively evaluated based on the parameter thus obtained. Note that the a is the value not dependent on the change of a total blood quantity of the capillary vascular system caused by an arterial constriction, and how to obtain the a will be explained hereafter.

First, in order to obtain the parameter for evaluating the numeric value of the blood viscosity, an electric circuit model of a vascular system is considered first. When the vascular system is formed into the electric circuit model, it appears that a voltage V corresponds to a vascular pressure P, and a current I corresponds to a blood flow rate S.

When the change of the flow is late, an artery model is formed as shown in Fig.6.

In this case, the resistance to a blood stream (vascular resistance) $R_0$ is expressed by formula (1):

$$R_0 = \frac{8\mu}{\pi R^4} \qquad \left[\frac{Pa\,s}{m^4}\right] \qquad \cdots (1)$$

A blood reactance $L_0$ is expressed by formula (2):

$$L_0 = \frac{4}{3}\frac{\rho}{\pi R^2} \qquad \cdots (2)$$

A capacitance of a vascular system (a blood quantity stored in a blood vessel) C is expressed by formula (3):

$$C = \frac{2\pi R^3(1-\sigma^2)}{Eh} \qquad \cdots (3)$$

Here, the designation marks indicate as follows:

$\rho$ :     mass of blood
$\mu$ :     viscosity ratio of blood
$\sigma$ :     poisson ratio of vascular wall
R :     inner diameter of blood vessel
E :     young's modulus of vascular wall

The formula (1) showing the resistance $R_0$ to blood flow expresses that the greater the vascular radius R is, the smaller the resistance is, and the greater the viscosity of the blood is, the greater the resistance becomes. Generally, the formula (1) is called the vascular resistance, which is dependent on the blood viscosity, because a viscosity $\mu$ of the blood is included in the formula, therefore is exactly called the resistance to blood flow here, in the meaning that the resistance occurs when the blood flows.

Next, a vein system is considered.

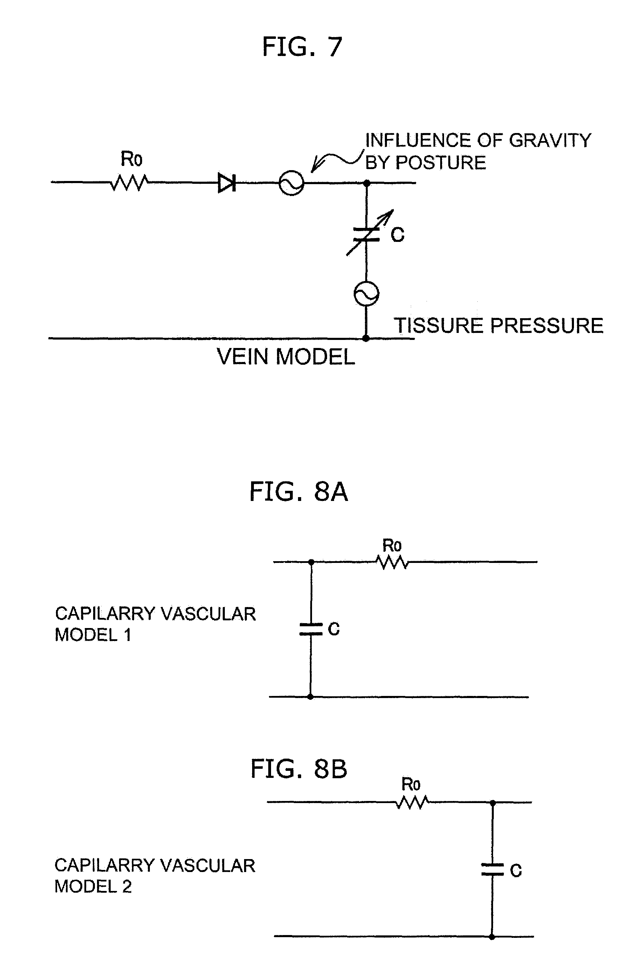

There is a vein valve in the vein, in which a steady-state flow flows, for preventing a back-flow. Therefore, by forming the vein valve into a model by diode, an electric equivalent circuit thereof is formed as shown in Fig.7. In this model, the influence of a tissue pressure and the influence of gravity by posture are included as elements. Here, the $R_0$ is the resistance to blood flow (vascular resistance) and expressed by the following formula (4):

$$R_0 = \frac{8\mu}{\pi R^4} \qquad \cdots (4)$$

Next, the capillary vascular system is considered.

The capillary blood vessel is the blood vessel formed of a layer of a vascular endothelial cell, having neither shrinkable muscular tissue like the artery, nor vein valve which can be formed into a model as an ideal diode like the vein system. Therefore, an equivalent circuit as shown in Fig.8 in which the vein model is deformed, can be considered to be the model of the capillary vascular system. In this case, two models of Fig.8A and Fig. 8B can be considered, wherein the model of Fig.8A is used at the time of the blood outflow from the capillary vascular system, and the model of Fig. 8B is used at the time of the blood inflow into the capillary vascular system.

The resistance to blood flow (vascular resistance) $R_0$ of Fig.8 is the same as that of the vein system, which is expressed by the following formula (5):

$$R_0 = \frac{8\mu}{\pi R^4} \qquad \left[\frac{Pa\, s}{m^4}\right] \qquad \cdots (5)$$

Here, a blood quantity Q stored in the capillary blood vessel can be approximated by the following formula (6) by using the vascular radius R.

$$Q = n\, \pi\, l_m\, R^2 \qquad [m^3] \qquad \cdots (6)$$

Wherein, n indicates the number of the capillary blood vessels to be the object, and 1m indicates an average length of the capillary blood vessel.

When the formula (6) is substituted to the formula (5), the formula (7) is obtained as follows:

$$R_0 = \frac{8\pi n^2 l_m{}^2 \mu}{Q^2} \qquad \cdots (7)$$

Here, it should be noted that the resistance to blood flow is proportional to a blood viscosity $\mu$, and the resistance to blood flow itself is dependent on the total blood quantity stored in the blood vessel. Specifically, when taking it consideration that a constant external pressure is applied to the blood vessel, and internal blood flows out to the vein system, the resistance R to blood flow is increased in accordance with the volume of a total flow rate Q, so as to suppress a flowing out blood quantity.

Next, blood kinetics when applying the oppression is considered.

Here, the case of applying a higher oppression than the maximum blood pressure of the artery to the cuff is considered. At this time, in regards to the artery, the internal blood is released at a high speed through a low vascular resistance of the artery (the radius of the artery is sufficiently larger than the vascular radius of the capillary vascular system, thereby decreasing the resistance to blood flow) . The similar phenomenon occurs to the vein system, and the internal blood flows out through the low resistance to blood flow of the vein system simultaneously with applying pressure.

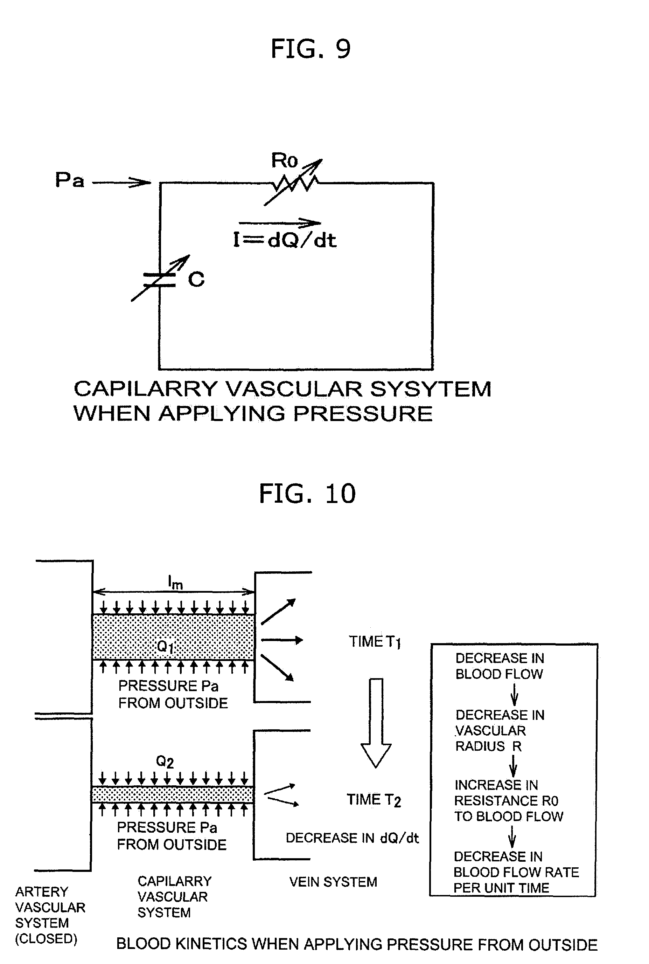

However, the blood stored in the capillary blood vessel receives the same pressure as the pressure applied from the outside, and is liable to flow out to the vein system. However, due to a high resistance to blood flow of the capillary vascular system, the blood flows out slowly. In addition, the radius R of the capillary vascular system is reduced, simultaneously with the outflow of the blood in the capillary blood vessel. Therefore, the resistance to blood flow becomes greater (see the aforementioned formula (5)), thereby making the blood outflow further slow. The equivalent electric circuit at this time is shown in Fig.9, because the resistance to blood flow of the vein system is sufficiently smaller than that of the capillary vascular system.

Here, the designation marks indicate as follows:

C : Capacity in the capillary blood vessel (electric charge Q stored therein corresponds to the quantity of blood)
Ro : Resistance to blood flow (see formula (7))
Pa : Pressure gradient applied from outside (unit s Pa/m)

The quantity of blood (current) I flowing out to the outside from the capillary vascular system is expressed by formula (8):

$$I = \frac{P_a}{R} = \frac{P_a}{8\pi n^2 l_m^2 \mu} Q^2 \qquad \left[m^3/s\right] \qquad \cdots (8)$$

When I = dQ/dt is taken into consideration, formula (9) can be obtained as a differential equation satisfying the quantity of blood Q stored in the capillary vascular system. Specifically, after applying the pressure, the quantity of blood Q in the capillary blood vessel is required to satisfy the differential equation as follows:

$$\frac{dQ}{dt} = -\alpha Q^2 \qquad \cdots (9)$$

wherein, a is expressed by formula (10):

$$\alpha = \frac{P_a}{8\pi n^2 l_m^2 \mu} \qquad \left[\frac{Pa/m}{m^2 \, Pa \, s} = \frac{1}{m^3 \, s}\right] \qquad \cdots (10)$$

A physical meaning of the differential equation is shown in Fig.10.
Fig.10 shows that when a pressure Pa is received from the outside at time T1, the outflow of the blood in the blood vessel occurs due to the pressure, thereby reducing a vascular radius R, to cause the increase in the resistance to blood flow Ro, resulting in the decrease in the blood flow rate (dQ/dt) per unit time.
Next, the relation between the quantity of blood Q in the capillary blood vessel, and a light reception intensity IR is considered.
The intensity of the light observed by a light detector (light receiving element) passing through the capillary blood vessel and placed on the surface of the skin again is expressed by formula (11):

$$I_R = K \, A_B \, A_T \, I_T \qquad \cdots (11)$$

wherein $A_B$ and AT show an attenuation of the light in the blood and the attenuation of the light in the tissue, respectively. Also, IT is the intensity of an incident light, and K is a coefficient. When the time change of the formula (11) is considered, the following formula (12) is obtained:

$$\frac{\partial I_R}{\partial t} = K \, A_T \, I_T \, \frac{\partial A_B}{\partial t} \qquad \cdots (12)$$

When the following formula (13) is approximately obtained between the attenuation of the blood and the quantity of blood, formula (13) is obtained:

$$\frac{\partial A_B}{\partial t} = -\beta \frac{\partial Q}{\partial t} \quad \cdots (1\,3)$$

As a relational expression between the light reception intensity and the quantity of blood, the following formula (14) is obtained:

$$\frac{\partial I_R}{\partial t} = -K\,\beta\,A_T\,I_T\,\frac{\partial Q}{\partial t} \quad \cdots (1\,4)$$

The aforementioned differential equation (9) satisfied by the quantity of blood Q in the capillary blood vessel can be analytically solved. First, both sides are divided by $Q^2$, and when a differentiation of Q is defined as Q', the following formula (15) is obtained:

$$\frac{Q'}{Q^2} = -\alpha \quad \cdots (1\,5)$$

This formula can be deformed into the following formula (16):

$$\left(\frac{1}{Q}\right)' = \alpha \quad \cdots (1\,6)$$

and from this formula, the following formula (17) can be obtained:

$$Q = \frac{1}{\alpha\,t + C} \quad \cdots (1\,7)$$

wherein C is an integral constant, and when an initial blood flow rate is defined as Qo when satisfying t = 0, the relation is expressed by C = 1/Qo.

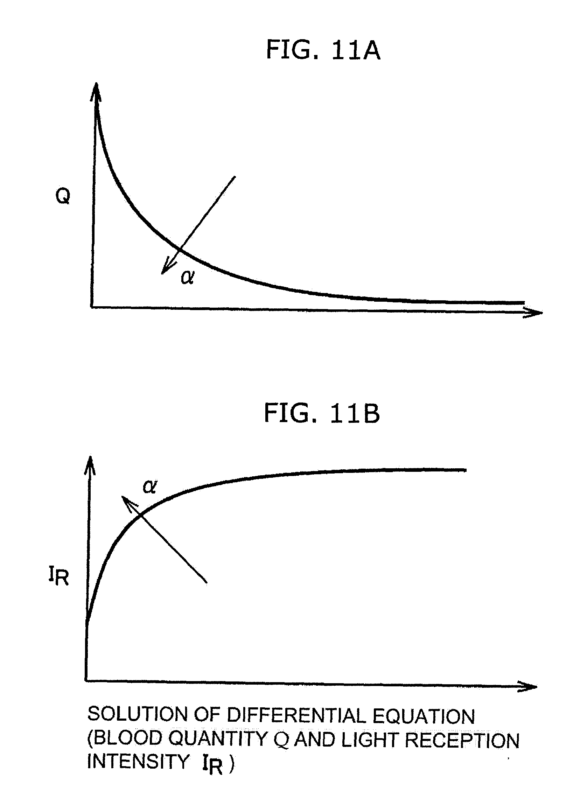

Q and a light reception intensity $I_R$ observed at this time is shown in Fig.11.

a becomes a parameter for determining a function system. However, as being clarified from the formula (10), the parameter a changes in proportion to an inverse number of the viscosity $\mu$ of the blood, and this reveals that the viscosity of the blood can be evaluated by estimating the parameter a from the measurement data of the light reception quantity.

Next, the physical meaning of the parameter a is considered. The parameter a of the formula (9), which is a motion equation expressing the change of the quantity of blood when the pressure is applied, is obtained by formula (10):

$$\alpha = \frac{P_a}{8\pi n^2 l_m{}^2 \mu} \quad \cdots (1\,0)$$

As clarified from the differential equation of the formula (9), this parameter expresses "the speed of reduction in the quantity of blood Q (due to application of the pressure). Next, the factor of determining this parameter is listed below.

1. Viscosity of blood $\mu$ :

When the viscosity of blood is great and the fluidity is lost, the value of the a becomes smaller.

Meanwhile, the smaller the viscosity is (the greater the fluidity of the blood is), the greater the a becomes.

2. n and 1m :

Here, a total volume of the blood quantity (total quantity of the blood) of the capillary blood vessel before applying pressure is defined as Qo, the relation becomes as the following formula (18):

$$Q_0 = \pi R_I^2 n l_m \quad \cdots (1\,8)$$

wherein $R_1$ is the vascular radius before applying pressure, and therefore the following formula (19) is obtained:

$$n l_m = \frac{Q_0}{\pi R_I^2} \quad \cdots (1\,9)$$

wherein, n and $l_m$ are parameters corresponding to a total extension of the blood vessel in the capillary blood vessel, and is not dependent on the total quantity of the blood of the capillary vascular system (as can be seen from the formula (19) wherein n and $l_m$ are divided by the term of the inner diameter). Namely, n and lm are the parameter dependent on the area where the pressure is applied.

3. Applying pressure Pa :

When the applying pressure becomes larger, the a becomes larger accordingly. In order to eliminate the influence of the Pa, a constant pressure may be applied, or the value normalized by the Pa in place of the a, which is expressed by the following formula (20), may be used:

$$\alpha_N = \alpha / P_a \quad \cdots (2\,0)$$

Next, an estimation method of the a from a light reception intensity $I_R$ observed when applying pressure is considered.

When the total quantity of blood Q is expressed by the formula (17), and the waveform is expressed by Fig. 11, the light reception intensity $I_R$ is expressed by the following formula (21):

$$I_r = I_{R,m} - \frac{1}{\alpha t + \frac{1}{I_{R,m}}} \quad \cdots (2\,1)$$

wherein the $I_R$,m is the maximum value of the light reception intensity.

When the formula (21) is differentiated by the time, the following formula (22) is obtained:

$$\frac{\partial I_R}{\partial t} = \frac{\alpha}{\left(\alpha t + \frac{1}{I_{R,m}}\right)^2} \quad \cdots (2\,2)$$

and the value when satisfying t = 0, which is the time of a differentiated value, is expressed by the following formula (23):

$$\frac{\partial I_R}{\partial t}\Big|_{t=0} = \alpha I_{R,m}{}^2 = K_0 \qquad \cdots (2 3)$$

and the value when satisfying t = 1 (sec) is expressed by the following formula (24):

$$\frac{\partial I_R}{\partial t}\Big|_{t=\mathrm{sec}} = \frac{\alpha}{\left(\alpha + \dfrac{1}{I_{R,m}}\right)^2} = \frac{\alpha I_{R,m}{}^2}{(\alpha I_{R,m}+1)^2} = K_1 \qquad \cdots (2 4)$$

In addition, from the formula (22), when the $I_{R,m}$ is normalized as 1, the following formula (25) is obtained:

$$\alpha = \frac{\partial I_R}{\partial t}\Big|_{t=0} \qquad \cdots (2 5)$$

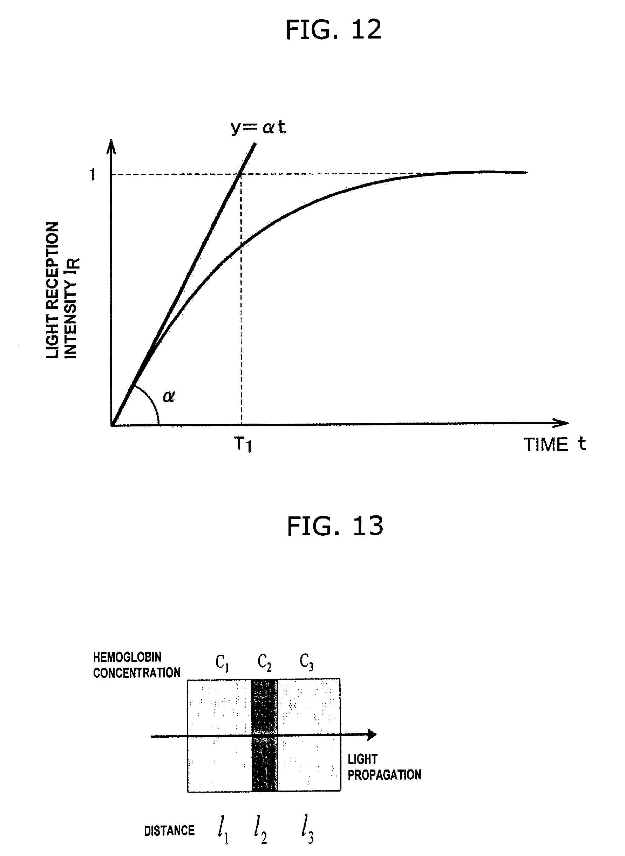

which is equal to an initial gradient obtained by normalizing by the maximum value of the light reception intensity.
As shown in the formula (25), the a is a differential coefficient satisfying t = 0 when the maximum value of the light reception intensity is normalized as 1, and this is shown in Fig.12.
Here, the time $T_1$ when linear line y =at becomes y = 1 is expressed by the following formula (26):

$$T_1 = \frac{1}{\alpha} \qquad \cdots (2 6)$$

wherein the $T_1$ also becomes the parameter related to the blood viscosity. Namely, the $T_1$ is the time required so that the whole quantity of the blood flows out when an initial blood outflow speed (= a) is assumed to continue at a constant value, and when a definition formula of the a is used, the T1 is expressed by the following formula (27):

$$T_1 = \frac{8\pi n^2 l_m{}^2}{P_a}\mu \qquad (\mathrm{sec}) \qquad \cdots (2 7)$$

which is the parameter proportional to the blood viscosity $\mu$.
In addition, the differential value satisfying the time t = n (sec) is obtained by the following formula (28):

$$\frac{\partial I_R}{\partial t}\Big|_{t=n\,\mathrm{sec}} = \frac{\alpha I_{R,m}{}^2}{(n I_{R,m}\alpha +1)^2} \qquad \cdots (2 8)$$

When the formula (23) is taken into consideration, the formula (29) is obtained:

$$\alpha = \frac{\left(\sqrt{K_0}-\sqrt{K_n}\right)^2}{n^2 K_0 K_n} \qquad \cdots (2 9)$$

As can be found from this formula (29), even when the maximum value $I_{R,m}$ of the light reception intensity can not be measured, when the initial differential value $K_o$ and a differential value Kn satisfying an arbitrary time t = n are found, the a can be obtained from these values, and from the a thus obtained, the viscosity of the blood can be evaluated.

Next, the fluidity evaluation method of the blood according to another embodiment will be explained.

The fluidity evaluation method of the blood according to this embodiment is characterized by calculating a hemoglobin concentration (ratio of the quantity of the hemoglobin when the whole tissue is defined as 1, taking the value from 0 to 1) in the tissue of the part where the oppression is applied from the data of the light reception intensity of the light receiving element 22, and evaluating the fluidity of the blood based on the time change of the hemoglobin concentration.

By way of an example of the evaluation method, the viscosity of the blood, which is one element of the fluidity of the blood, is evaluated, based on the time T (= $t_2$ - t1) required for reducing the hemoglobin concentration from the stage of a first predetermined value (such as 2% or 1%) which is already reduced and is a sufficiently smaller value than that before applying pressure, up to a second predetermined value (1% or 0.5% of a half of a first predetermined value) which is a further smaller value than the first predetermined value.

Also, in the next stage, a blood viscosity evaluation parameter is calculated from the data of the light reception intensity of the light receiving element 22, and the relation between the blood viscosity evaluation parameter and the hemoglobin concentration is displayed in a graph, with the blood viscosity evaluation parameter taken on the ordinate axis, and the hemoglobin concentration taken on the abscissa axis.

Generally, the viscosity of the blood is considered to be increased due to the deterioration in the red blood cell deformation efficiency and the aggregation phenomenon of the red blood cell, the increase in a hematocrit value (volume % of the red blood cell) and the change in viscoelastic characteristics of a white blood cell and a blood plasma. In addition, the blood has so-called rheological characters that the viscosity is significantly decreased when a shear rate becomes large.

By quantitatively measuring the hemoglobin concentration in the capillary blood vessel of the blood having the aforementioned characters, it appears that the viscosity of the blood can be estimated therefrom. There are a plurality of capillary blood vessels on the surface of the skin of a human being, and the capillary blood vessels control a direct blood circulation to skin cells. When the pressure of more than the blood pressure is applied to the capillary blood vessels on the surface of the skin, the blood flows out from the capillary blood vessels, and the quantity of the blood in the capillary blood vessels under the skin to which the pressure is applied is gradually decreased. A blood outflow phenomenon from the capillary blood vessels occurs when the vascular diameter becomes smaller by applying pressure, thereby pushing out the blood.

However, since the vascular diameter of the capillary blood vessel is significantly small, the quantity of the blood flowing out is limited, and slowly flows out to the vascular system to which no other pressure is applied. Also, as described above, when the vascular diameter becomes minute, the change in the viscosity such as the aggregation of the red blood cell and the deterioration in the deformation efficiency of the red blood cell occurs. Then, under such an influence, an outflow quantity of the blood from the capillary blood vessel is decreased, and it is decreased further with the passage of the time of a pressure time. In other words, by observing a process of the outflow of the blood of the capillary blood vessel due to applying pressure, the viscosity of the blood can be measured.

When the outflow of the blood is optically measured from the outside of the skin, the hemoglobin and other living body constituting materials in the living body largely absorbs the visible light, and the visible light hardly transmits the living body. However, by applying pressure to the skin, the blood flows out from the capillary blood vessel. Along with this, the blood (hemoglobin) is decreased, and the light absorption in the living body is decreased compared with that before applying pressure. Specifically, by applying pressure, the blood in the capillary blood vessel flows out, and the light transmits the living body along with the decrease in the quantity of the blood (quantity of the hemoglobin).

In addition, in regards to the wavelength of the light, the light with short wavelength hardly transmits the living body. Specifically, it can be considered that to observe the light with short wavelength with which the living body is irradiated, is to observe the light propagated through the capillary blood vessel directly under the skin. By utilizing this principle, the condition of the outflow of the blood in the capillary blood vessel due to applying pressure can be observed, and the hemoglobin concentration can be measured therefrom.

Next, determination of the hemoglobin concentration is considered.

Originally, the incident light reaches the detector, while a multiple scattering is performed in the tissue of the living body. However, for simplification here, the incident light is considered to reach the detector through some optical path. Here, this equivalent optical path length is defined as 1 (transmitting transducer). The optical path is considered to be always constant, irrespective of whether or not the blood is present in the capillary blood vessel. According to Lambert-Beer's law, the attenuation by the hemoglobin in the blood is given by the formula expressed by:

$$A_B = \exp(-\varepsilon c \ell) \qquad (41)$$

wherein e : Photoabsorption coefficient of the hemoglobin by the wavelength of the light (unit : mm$^{-1}$)

c : Hemoglobin concentration in the tissue (which is the ratio and a non-dimensional quantity taking the value from 0 to 1)

The attenuation of the light in the tissue of the living body is defined as AT. This attenuation includes the attenuation by a blood component remaining in the tissue even after applying pressure for a sufficient period of time, in addition to the attenuation of the tissue. Also, a detection efficiency of the detector is defined as K.

At this time, by whether or not applying the pressure to the blood of the capillary blood vessel, the output $I_r$ of the detector is expressed as follows:

(a) Before applying the pressure (in a state where the blood is present), the output $I_{rB}$ of the detector is expressed by formula (42):

$$I_{rB} = K I_0 A_T \exp(-\varepsilon c \ell) + I_D \qquad (42)$$

wherein the $I_D$ is a direct propagation light (including outside light) between a light source - detector.

(b) After a sufficient period of time passes after applying the pressure (or after a sufficient period of time passes, the value is estimated by a suitable bias estimation method), the output $I_{rT}$ of the detector is expressed by formula (43):

$$I_{rT} = K I_0 A_T + I_D \qquad (43)$$

When a direct light $I_D$ can be measured, the values obtained by deducting the direct light $I_D$ from the formulas (42) and (43) are expressed by the formulas:

$$I'_{rB} = I_{rB} - I_D = K I_0 A_T \exp(-\varepsilon c \ell) \qquad (44)$$

$$I'_{rT} = I_{rT} - I_D = K I_0 A_T \qquad (45)$$

When logarithms of the values of the formulas (44) and (45) are taken, the formulas (46) and (47) are obtained expressed by:

$$\ln(I'_{rB}) = \ln(K I_0 A_T) - \varepsilon c \ell \qquad (46)$$

$$\ln(I'_{rT}) = \ln(K I_0 A_T) \qquad (47)$$

Therefore, the formula (48) is obtained:

$$\varepsilon c \ell = \ln(I'_{rT}) - \ln(I'_{rB}) = \ln\left(\frac{I'_{rT}}{I'_{rB}}\right) \qquad (48)$$

Thus, with the equivalent light path length 1 and the photoabsorption coefficient e already known, the hemoglobin concentration c is expressed by the formula (49):

$$c = \frac{\ln\left(\dfrac{I'_{rT}}{I'_{rB}}\right)}{\varepsilon \ell} \qquad (49)$$

For example, the quantity of the hemoglobin in the tissue is decreased along with the decrease in the quantity of the blood just after applying pressure. However, when the hemoglobin concentration $c_i$ at an arbitrary time point becomes ci, and the light reception intensity of the light receiving element at this time point is defined as $I_rB_i$, the hemoglobin concentration $c_i$ at the arbitrary time point is estimated by the following formula. However, here, it appears that there is no influence of the direct propagation light $I_D$.

$$c_i = \frac{1}{\varepsilon l}\ln\left(\frac{I_{rT}}{I_{rBi}}\right) \qquad (50)$$

The formula (50) is a general formula measuring the hemoglobin concentration, and the hemoglobin concentration $c_i$ along the propagation path of the light can be estimated.

Generally, it appears that layers of different hemoglobin concentrations such as a surface skin, a true skin, and a subcutaneous tissue are laid on each other on the propagation path of the light. Therefore, when this part is included, as shown in Fig.13, and the light propagates a plurality of layers with different hemoglobin concentration, the light reception intensity, with the layer (thickness $l_i$) of the hemoglobin concentration $c_i$ laid in series on the light propagation path, is expressed by formula (51):

$$I_{rB} = KI_0 A_{r'} \exp\left(-\sum_i \varepsilon_i c_i l_i\right) \qquad (51)$$

wherein ei is the photoabsorption coefficient of a i-th layer, $c_i$ is the hemoglobin concentration of the i-th layer, and $l_i$ is the thickness of the i-th layer.

In this case also, the light intensity when passing through a precedent layer after applying pressure and after a sufficient period of time passes (actually, after estimating a final value (bias value) of the light intensity) is expressed by formula (52)

$$\sum_i \varepsilon_i c_i l_i = \ln\left(\frac{I_{rT}}{I_{rB}}\right) \qquad (52)$$

and a weighting average value of the quantity of the hemoglobin can be estimated.

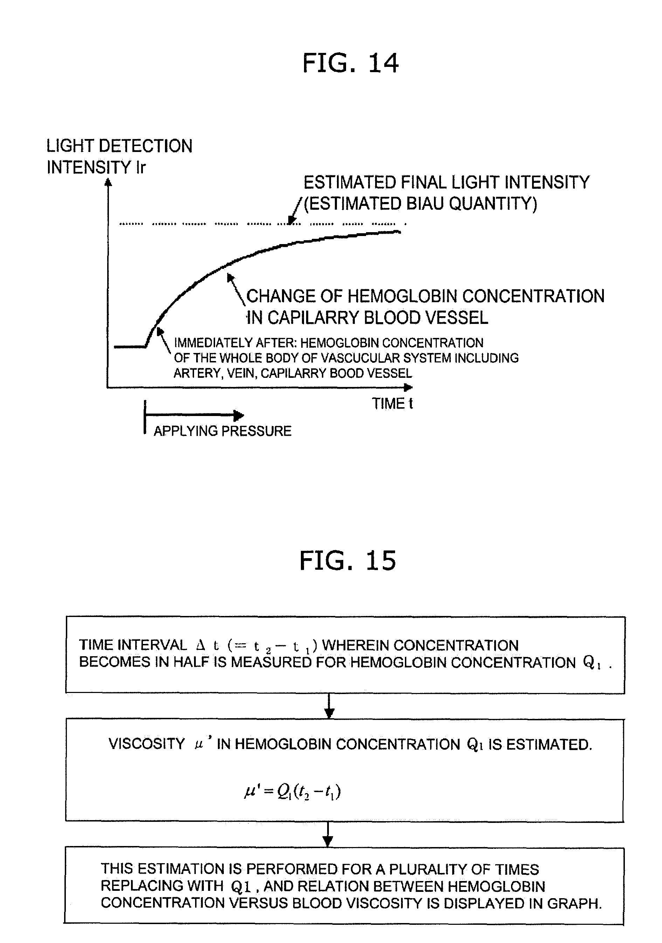

However, the skin has a layer structure of the surface skin, the true skin, and the subcutaneous tissue, and the blood of the artery and the vein with small vascular resistance flows out at a high speed immediately after a certain period of time passes. Therefore, it is proper to consider that the quantity of the hemoglobin estimated by the formula (52) after a certain period of time passes is dependent on the capillary blood vessel. This is outlined in Fig.14.

Next, explanation will be given to a method of estimating the viscosity of the blood from a time interval wherein the hemoglobin concentration is decreased by a predetermined quantity.

As the estimation method of the blood viscosity utilizing the light reception intensity, in the aforementioned embodiment, the method of obtaining the blood viscosity from a time differential of the light reception intensity (= quantity of blood) Q is given as an example. However, in this embodiment, the blood viscosity is obtained from the time interval wherein the hemoglobin concentration is decreased.

The time change of the quantity of the blood Q (which is replaced with the hemoglobin concentration, with the hematocrit value considered to be fixed) is expressed by formula (61):

$$Q = \frac{1}{\alpha t + \frac{1}{Q_0}} = \frac{Q_0}{\alpha Q_0 t + 1} \qquad (61)$$

wherein,

$Q_0$ : Quantity of blood just before applying pressure (considered to be hemoglobin concentration)

$$\alpha = \frac{P_a}{8\pi n^2 \ell_m^2 \mu} = K \frac{1}{\mu}$$

$\mu$ : Blood viscosity

When the hemoglobin concentration Q1 is obtained at time t1, and the hemoglobin concentration Q2 is obtained at time t2, formula (62) is obtained from the formula (61):

$$Q_1 = \frac{Q_0}{\alpha Q_0 t_1 + 1}$$

$$Q_2 = \frac{Q_0}{\alpha Q_0 t_2 + 1} \qquad (62)$$

From this formula, the formula (63) is obtained:

$$t_1 = \frac{Q_0 - Q_1}{\alpha Q_0 Q_1}$$

$$t_2 = \frac{Q_0 - Q_2}{\alpha Q_0 Q_2} \qquad (63)$$

Therefore the formula (64) is obtained:

$$t_2 - t_1 = \frac{1}{\alpha}\left(\frac{Q_0 - Q_2}{Q_0 Q_2} - \frac{Q_0 - Q_1}{Q_0 Q_1}\right) = \frac{1}{\alpha}\left(\frac{Q_1 - Q_2}{Q_1 Q_2}\right) \qquad (64)$$

Specifically, the parameter a relating to the inverse number of the viscosity $\mu$ is expressed by formula (65):

$$\alpha = \frac{Q_1 - Q_2}{Q_1 Q_2} \cdot \frac{1}{t_2 - t_1} \qquad (65)$$

Alternately, as a parameter $\mu'$ proportional to a viscosity $\mu$, formula (66) is obtained:

$$\mu' = \frac{1}{\alpha} = \frac{Q_1 Q_2}{Q_1 - Q_2}(t_2 - t_1) \qquad (66)$$

Here, when the viscosity related parameter $\mu'$ is considered. The unit of the parameter $\mu'$ becomes %/sec, with the hemoglobin concentration (%) defined as Q, and the time defined as t. Thus, the blood viscosity is expressed between the hemoglobin concentrations $Q_1$ and $Q_2$, and the greater the value is, the greater the viscosity is. For example, when $Q_2 = 0.5Q_1$, the formula (66) is expressed by formula (67):

$$\mu' = Q_1(t_2 - t_1) \qquad (67)$$

Specifically, when the hemoglobin concentration is selected to be 2% as $Q_1$, the viscosity of the blood with hemoglobin concentration of 2% in the formula (67) can be estimated from measuring the time interval ($t_2 - t_1$) wherein the hemoglobin concentration is decreased to 1%, namely, is decreased in half.
In the same way, when the hemoglobin concentration is selected to be 1% as $Q_1$, the viscosity of the blood with hemoglobin concentration of 1% in the formula (67) can be estimated from measuring the time interval ($t_2 - t_1$) wherein the hemoglobin concentrations is decreased to 0.5%. It can be said that the longer the time wherein the hemoglobin concentration is decreased in half, the higher the viscosity is.
A flowchart for estimating a blood viscosity related parameter $\mu'$ is shown in Fig.15.
First, in a first step, in regards to the hemoglobin concentration $Q_1$, the time interval ($t_2 - t_1$) wherein the concentration is decreased in half is measured. Next, in a second step, the viscosity $\mu'$ of the blood with hemoglobin concentration $Q_1$ is estimated. Then, in a third step, the blood viscosity is estimated in the same way for a plurality of times while changing the hemoglobin concentration $Q_1$, and the relation between the "hemoglobin concentration" versus "blood viscosity parameter" is shown in a graph.
Note that the time ($t_2 - t_1$) wherein the hemoglobin concentration is decreased in half 0.5 $Q_1$ is expressed by formula (71):

$$(t_2 - t_1) = \frac{1}{\alpha Q_1} \qquad (71)$$

Here, the meaning of the ($t_2 - t_1$) is considered.
When the hemoglobin concentration is $Q_1$ at the time of t = 0, the time change is expressed by formula (72):

$$Q = \frac{Q_1}{\alpha Q_1 t + 1} \qquad (72)$$

The derivative at t is expressed by formula (73):

$$\frac{dQ}{dt} = -\frac{\alpha Q_1^2}{(\alpha Q_1 t + 1)^2} \qquad (73)$$

The value of differentiation at t = 0 (Namely, the quantity of the hemoglobin is Q1) is expressed by formula (74):

$$\frac{dQ}{dt}\Big|_{t=0} = -\alpha Q_1^2 \qquad (74)$$

A tangent line of the quantity of the hemoglobin Q, having the gradient of the formula (74) and having Q1 at t = 0 is considered to be expressed by formula (75):

$$Q = -\alpha Q_1^2 t + Q_1 \qquad (75)$$

The time T is obtained so that the tangent line satisfies Q = 0:

$$T = \frac{1}{\alpha Q_1} = (t_2 - t_1) \qquad (76)$$

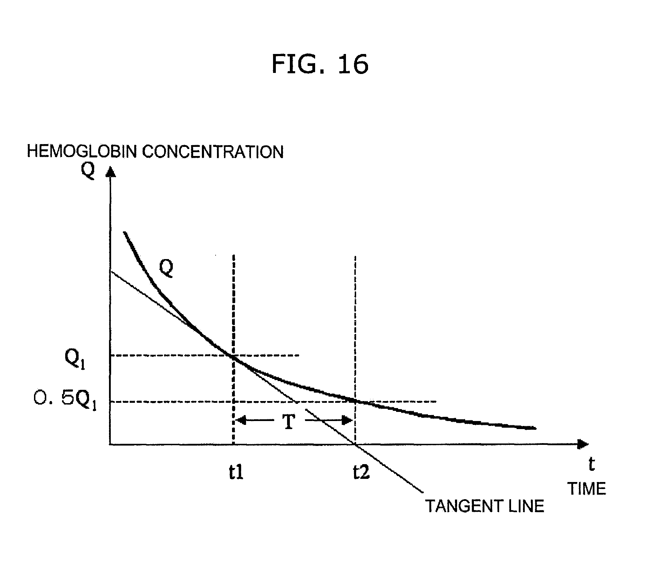

The relation of this formula is shown in Fig.16.

Therefore, the time $(t_2 - t_1)$ wherein the hemoglobin concentration becomes in half has the following three meanings:

(1) $(t_2 - t_1)$ is the time wherein the hemoglobin concentration Q becomes in half (original meaning).
(2) significance of $(t_2 - t_1)$ with $\alpha$ or viscosity parameter $\mu'$.

$$\alpha = \frac{1}{Q_1(t_2 - t_1)} \qquad (77)$$

$$\mu' = \frac{1}{\alpha} = Q_1(t_2 - t_1) \qquad (78)$$

(3) T (= t2 - t1) is the time wherein the tangent line satisfies Q = 0, with the hemoglobin concentration Q1. Wherein, T is the time required for the whole blood to flow out, when the outflow of the blood is assumed to continue constantly (an outflow speed of the blood satisfying $Q = Q_1$ continues constantly).

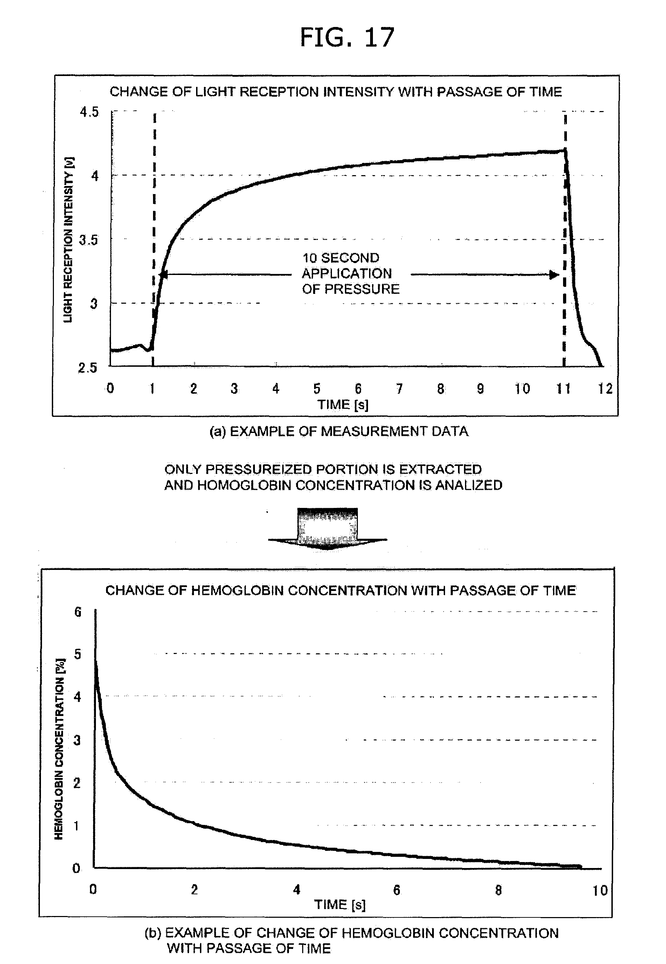

Next, an actual measurement result is considered.
An actual measurement was performed as follows. First, the pressure was applied after one second from starting the measurement, and then after 10 seconds, the pressure was released and the measurement was completed. Fig. 17 shows an example of measurement data.
The flow of processing is as follows:

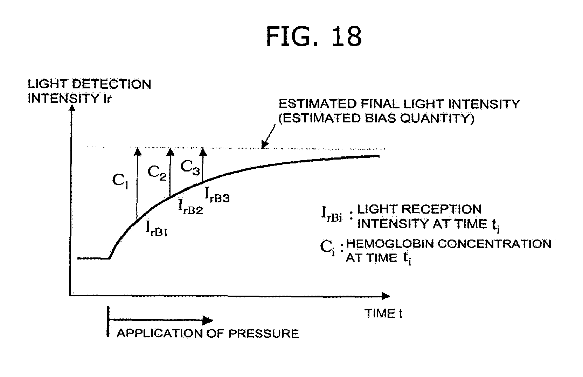

(1) Light reception intensity is measured.
(2) The light reception intensity with time set to be infinite is estimated (some point of the actual measurement value is considered to be a convergence value, and the difference between the convergence value and a temporary convergence value, with time set to be infinite, namely, a bias value in a model equation is obtained).
(3) The hemoglobin concentration is calculated (the hemoglobin concentration is calculated from the light reception intensity immediately after applying pressure and the light reception intensity after a sufficient time period passes after applying pressure. $C_1$, $C_2$, and $C_3$ of Fig.18 correspond to the hemoglobin concentration.
(4) The blood viscosity parameter is calculated (the blood viscosity parameter $\mu'$ ($\mu' = 1/\alpha$) is derived from the time wherein the hemoglobin concentration becomes in half).

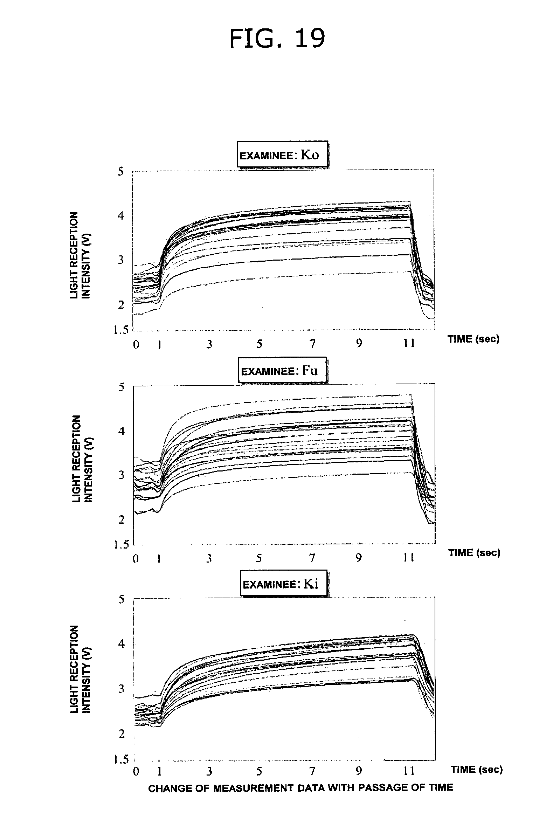

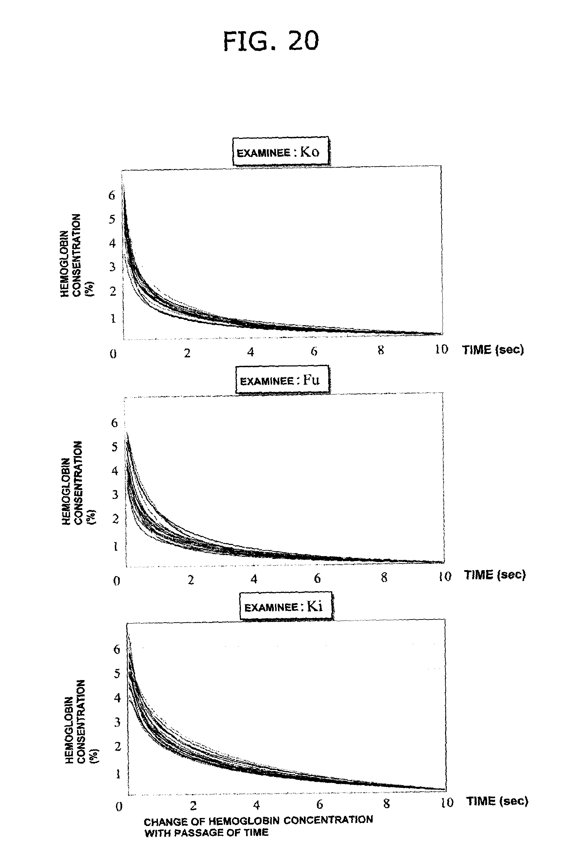

Fig.19 and Fig.20 show the result of an experiment conducted for a plurality of examinees. Here, four data of light receiving elements per one measurement is simultaneously collected, and this is performed for a plurality of times. Then, the result is shown in Figs. 19 and 20.

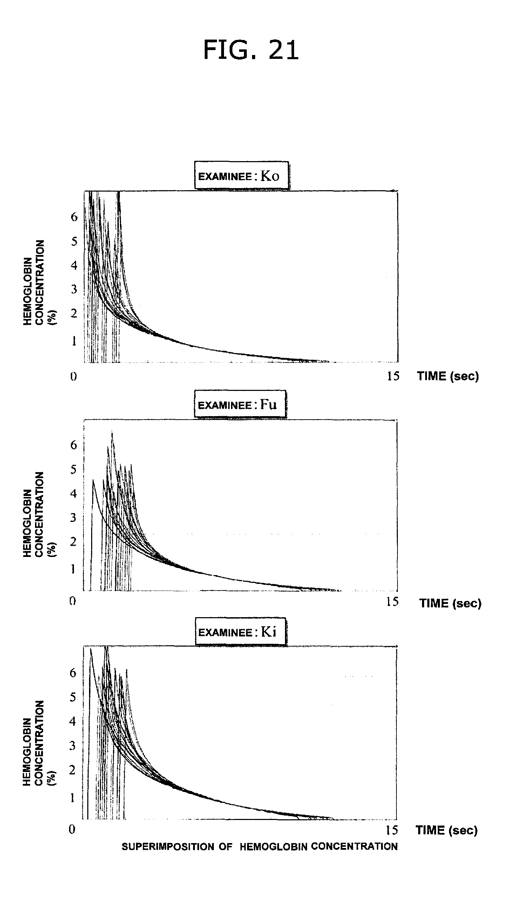

These data reveals large initial variations immediately after applying pressure. It appears that the initial variation is caused by the difference in each measurement condition having an affect on the hemoglobin concentration. When several seconds pass after applying pressure, the variation becomes small. Therefore, a converging phenomenon of a waveform is focused, when continuously applying pressure to the skin, irrespective of the initial variations. Fig.21 is a view of the hemoglobin concentration which is moved in a direction of time, superimposing each converging portion.

Here, the portion where the waveform is converged is focused, and the portion which is not easily affected by the blood pressure and AVR (artery - vein shunt) is selected from the model equation of the blood outflow in the capillary blood vessel, and the blood viscosity is evaluated. The vascular resistance is high in the capillary blood vessel, and therefore even when the pressure is applied, the blood flows out slowly, and a long time is required for closing the blood vessel.

When the hemoglobin concentration is large, the graph of a concentration change show a large variation under each condition. However, when the hemoglobin concentration is 2% or less, the graph is converged. Therefore, the change at the stage where the hemoglobin concentration becomes 2% or less is checked. This is because the variation immediately after applying pressure can be eliminated.

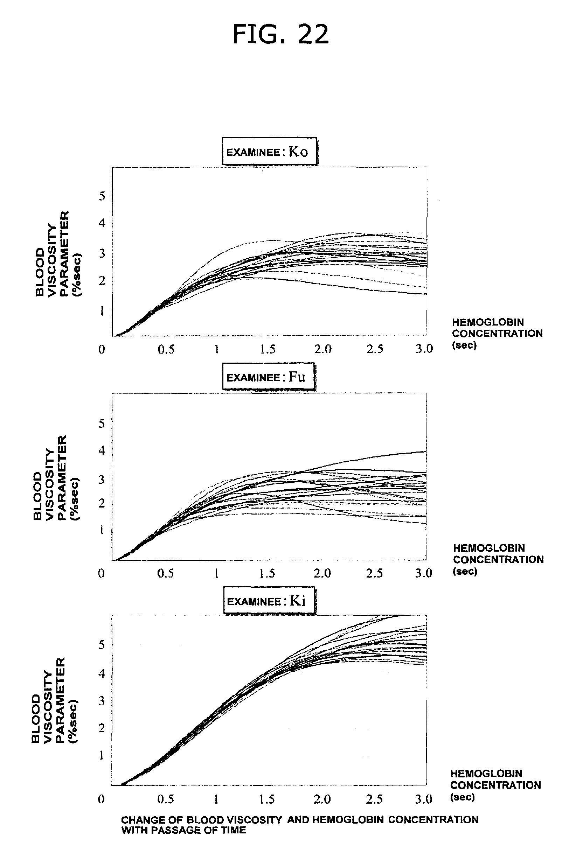

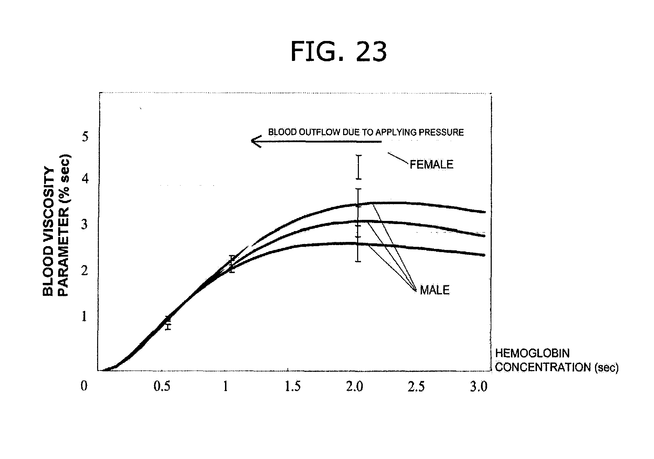

Fig.22 is a view showing the relation between the blood viscosity evaluation parameter and the hemoglobin concentration, with the blood viscosity evaluation parameter taken on the ordinate axis and the hemoglobin concentration taken on the abscissa axis. This reveals a different tendency for each examinee, and, the data of each examinee is averaged and grouped. This is shown in Fig.23 which reveals the different tendency between female and male.

In this way, by evaluating the viscosity of the blood based on the hemoglobin concentration, the following effect can be obtained. Specifically, for example, the hemoglobin concentration in the tissue changes with the passage of time after applying pressure (changes along with the blood outflow), and when the viscosity of the blood is evaluated based on the change at the stage where the hemoglobin concentration is large, it is possible to know a viscosity characteristic of the whole blood as a multi-phase flow particularly composed of the red blood cell and plasma (related to a so-called kinematic viscosity coefficient, and considered to be corresponding to the degree of a sticky condition of blood flow). Also, when the viscosity of the blood is evaluated based on the change at the stage where the hemoglobin concentration in the tissue becomes sufficiently small, it is possible to know the viscosity characteristic that reflects the change in the blood cell deformation efficiency caused by a sufficiently small diameter of the capillary blood vessel.

In addition, the hemoglobin concentration in the tissue can be called "the parameter determining a good or undesirable circulation of the blood to the capillary blood vessel". Therefore, by measuring (or estimating) the hemoglobin concentration, usability can be exhibited for the evaluation of a risk factor of bedsore (pressure sore) caused by a circulatory disorder to the capillary vascular system, a prevention of frostbite (including a mild symptom such as chilblain), and the evaluation of a skin regenerating property after external injury.

In addition, by evaluating the viscosity of the blood based on the time interval wherein the hemoglobin concentration is decreased in half, the viscosity evaluation of the blood can be performed in a short time at the stage where the blood sufficiently flows out from the capillary vascular system, that is, at the stage where there is no influence of the artery - vein shunt.

In addition, by displaying the relation between the blood viscosity evaluation parameter and the hemoglobin concentration in one graph as shown in Fig.23, only required level with no influence of the artery - vein shunt can be displayed, and the viscosity can be evaluated, irrespective of the initial quantity of the blood and the difference in elapsed time after applying pressure. Further, according to this graph, the rheological property of the blood (condition of changing the viscosity according to the vascular diameter, (corresponding to the quantity of the hemoglobin, here)) can be confirmed at a glance. In summary it is an object of the invention to easily evaluate a fluidity of blood with high sensitivity. By winding a pressure bag body 10 around the surface of a skin of a finger tip 100 or the like of an examinee, and applying an oppression thereto, the blood in a capillary blood vessel under the skin is flown out to the surroundings, and by irradiating the skin where the oppression is applied with light from a light emitting element 21, and measuring a degree of the light absorption as a light reception quantity of a light receiving element 22, thereby optically measuring the change of a quantity of blood in the capillary blood vessel, and obtaining the quantity of blood in the capillary blood vessel with passage of time after applying the oppression, the fluidity of the blood (mainly viscosity $\mu$) is evaluated.

**Claims**

1. A method of evaluating a fluidity of blood, comprising:

   applying an oppression on a surface of a skin of an examinee;
   measuring the change of a remaining blood quantity under the skin with passage of time caused by a blood outflow from the capillary blood vessel under the skin; and
evaluating the resistance to a blood flow by its measurement data.

2. A method of evaluating a fluidity of blood, comprising:

    making blood in a capillary blood vessel under the skin flow out to the surroundings by applying an oppression on a surface of a skin of a predetermined portion of an examinee;
    optically measuring the change of a blood quantity in the capillary blood vessel by measuring the degree of light absorption when the surface of a skin of a predetermined portion of an examinee is irradiated with light; and
    evaluating the fluidity of the blood by obtaining the change of the blood quantity in the capillary blood vessel with passage of time after the oppression is applied thereto.

3. The method of evaluating the fluidity of blood according to claim 2, wherein as an optical detector for measuring the degree of the light absorption when the skin of the predetermined portion is irradiated with light, combination of a light emitting element which irradiates the surface of the skin with light and a light receiving element which receives a returning light from under the skin is used, to set relative positions between the light emitting element and the light receiving element and the wavelength of an irradiating light, so that although the light reaches the capillary blood vessel under the skin, it does not reach a blood vessel such as an arteriole present in a location further deeper than the capillary blood vessel.

4. The method of evaluating the fluidity of blood according to claim 3, comprising:

    arranging the light emitting elements at a plurality of regular intervals;
    emitting the light from each light emitting element while shifting the time; thereby
    collecting a plurality of measurement data through the light receiving element; and
    evaluating a fluidity of the blood based on the data obtained by averaging the plurality of measurement data.

5. The method of evaluating the fluidity of blood according to any one of claims 2 to 4, comprising:

    calculating a blood viscosity evaluation parameter from the data of the change of the blood quantity with passage of time; and based on the parameter thus obtained,
    evaluating the viscosity of the blood.

6. The method of evaluating the fluidity of blood according to claim 3, comprising:

    calculating a hemoglobin concentration (which is a ratio of a quantity of hemoglobin when the whole tissue is defined as 1, and the values are varied from 0 to 1) in the tissue of a predetermined portion where the oppression is applied based on a light reception intensity; and
    evaluating the fluidity of the blood based on the change of the hemoglobin concentration with passage of time.

7. The method of evaluating the fluidity of blood according to claim 6, comprising:

    evaluating the viscosity of the blood, which is one element of the fluidity of the blood, based on a time wherein the hemoglobin concentration is decreased from a first predetermined value which is already reduced and sufficiently smaller than the value before applying the oppression, to a further smaller second predetermined value.

8. The method of evaluating the fluidity of blood according to claim 7, wherein the second predetermined value is about half of the first predetermined value.

9. The method of evaluating the fluidity of blood according to any one of claims 6 to 8, wherein a blood viscosity evaluation parameter is calculated from the data of a light reception intensity of the light receiving element, and the relation between the blood viscosity evaluation parameter and the hemoglobin concentration is shown in a graph.

10. An apparatus for evaluating a fluidity of blood, comprising:

    a pressurizer for applying oppression on the surface of a skin of an examinee;
    an optical detector for optically measuring a degree of a light absorption by irradiating the skin of a part where the oppression is applied with light;

a calculator for calculating the change of a blood quantity in a capillary blood vessel under the skin with passage of time by measurement data of the optical detector after applying pressure; and

a display device for displaying an evaluation content of the fluidity of the blood based on a calculation result by the calculator.

11. The apparatus for evaluating the fluidity of blood according to claim 10, comprising:

the pressurizer having a pressure bag body for applying an oppression force to the skin by introducing a pressure air; and

the optical detector having the combination of a light emitting element for irradiating the surface of a skin with light and a light receiving element for receiving a returning light from under the skin, wherein the light emitting element and the light receiving element are provided in a part touching on the skin by the pressure bag body or the part pushed against the skin by an expansion of the pressure bag body.

12. The apparatus for evaluating the fluidity of blood according to claim 11, wherein relative positions between the light emitting element and the light receiving element and a wavelength of an irradiating light are set so that although the light reaches a capillary blood vessel under the skin, it does not reach a blood vessel such as an arteriole present in a location further deeper than the capillary blood vessel.

# FIG. 1

21 LIGHT EMITTING ELEMENT    10 PRESSURE BAG BODY

100

22 LIGHT RECEIVING ELEMENT

ATMOSPHERIC AIR

| 23 | DRIVE CIRCUIT OF LIGHT EMITTING ELEMENT |
|---|---|

11    13

ELECTROMAGNETIC VALUE 1

| 24 | AMPLIFICATION CIRCUIT OF LIGHT RECEPTION SIGNAL |
|---|---|

12    14

ELECTROMAGNETIC VALUE 2

17

PRESSURE SENSOR

AIR TANK    15

| 25 | BLOOD VISCOSITY CALCULATION CIRCUIT |
|---|---|

AIR COMPRESSOR

16

| 26 | DISPLAY UNIT |
|---|---|

CONTROLLER

50

OPERATION KEY

51

# FIG. 2A

10 PRESSURE BAG BODY

100

21 LIGHT EMITTING ELEMENT

22 LIGHT RECEIVING ELEMENT

# FIG. 2B

21

22

21

10

# FIG. 3A

START OF
PRESSURE

CANCEL OF
PRESSURE

PRESSURE

150mmHg (EXAMPLE)

TIME ⟶

# FIG. 3B

LOW VISCOSITY

MEDIUM
VISCOSITY

LIGHT
RECEPTION
QUANTITY

HIGH
VISCOSITY

TIME ⟶

25

# FIG. 4

( a ) CHANGE OF LIGHT RECEPTION QUANTITY AT LOW BLOOD VISCOSITY

# FIG. 5

( a )CHANGE OF LIGHT RECEPTION QUANTITY AT HIGH BLOOD VISCOSITY

CANCEL OF PRESSURE

APPLICATION OF PRESSURE

ABOUT 3 SECONDS

1/10 SECONDS

# FIG. 6

ARTERY MODEL

# FIG. 7

INFLUENCE OF GRAVITY
BY POSTURE

R0

C

TISSURE PRESSURE

VEIN MODEL

# FIG. 8A

R0

C

CAPILARRY VASCULAR
MODEL 1

# FIG. 8B

R0

C

CAPILARRY VASCULAR
MODEL 2

# FIG. 9

$Pa \rightarrow$

$R_0$

$I = dQ/dt$

$C$

CAPILARRY VASCULAR SYSYTEM
WHEN APPLYING PRESSURE

# FIG. 10

$I_m$

$Q_1$

PRESSURE Pa
FROM OUTSIDE

$Q_2$

PRESSURE Pa
FROM OUTSIDE

TIME $T_1$

TIME $T_2$

DECREASE IN dQ/dt

DECREASE IN
BLOOD FLOW
↓
DECREASE IN
VASCULAR
RADIUS R
↓
INCREASE IN
RESISTANCE R0
TO BLOOD FLOW
↓
DECREASE IN
BLOOD FLOW RATE
PER UNIT TIME

ARTERY
VASCULAR
SYSTEM
(CLOSED)

CAPILARRY
VASCULAR
SYSTEM

VEIN SYSTEM

BLOOD KINETICS WHEN APPLYING PRESSURE FROM OUTSIDE

# FIG. 11A

# FIG. 11B

SOLUTION OF DIFFERENTIAL EQUATION
(BLOOD QUANTITY Q AND LIGHT RECEPTION
INTENSITY $I_R$ )

## FIG. 12

$y = \alpha t$

$\alpha$

1

LIGHT RECEPTION INTENSITY $I_R$

$T_1$

TIME $t$

## FIG. 13

HEMOGLOBIN CONCENTRATION

$C_1$ $C_2$ $C_3$

LIGHT PROPAGATION

DISTANCE $\quad l_1 \quad l_2 \quad l_3$

# FIG. 14

LIGHT DETECTION
INTENSITY Ir

ESTIMATED FINAL LIGHT INTENSITY
(ESTIMATED BIAU QUANTITY)

CHANGE OF HEMOGLOBIN CONCENTRATION
IN CAPILARRY BLOOD VESSEL

IMMEDIATELY AFTER: HEMOGLOBIN CONCENTRATION
OF THE WHOLE BODY OF VASCUCULAR SYSTEM INCLUDING
ARTERY, VEIN, CAPILARRY BOOD VESSEL

TIME t

APPLYING PRESSURE

# FIG. 15

TIME INTERVAL $\Delta t$ ($= t_2 - t_1$) WHEREIN CONCENTRATION
BECOMES IN HALF IS MEASURED FOR HEMOGLOBIN CONCENTRATION $Q_1$ .

VISCOSITY $\mu'$ IN HEMOGLOBIN CONCENTRATION $Q_1$ IS ESTIMATED.

$$\mu' = Q_1(t_2 - t_1)$$

THIS ESTIMATION IS PERFORMED FOR A PLURALITY OF TIMES
REPLACING WITH $Q1$ , AND RELATION BETWEEN HEMOGLOBIN
CONCENTRATION VERSUS BLOOD VISCOSITY IS DISPLAYED IN GRAPH.

# FIG. 16

HEMOGLOBIN CONCENTRATION

# FIG. 17

**CHANGE OF LIGHT RECEPTION INTENSITY WITH PASSAGE OF TIME**

10 SECOND
APPLICATION
OF PRESSURE

(a) EXAMPLE OF MEASUREMENT DATA

ONLY PRESSUREIZED PORTION IS EXTRACTED
AND HOMOGLOBIN CONCENTRATION IS ANALIZED

**CHANGE OF HEMOGLOBIN CONCENTRATION WITH PASSAGE OF TIME**

(b) EXAMPLE OF CHANGE OF HEMOGLOBIN CONCENTRATION
WITH PASSAGE OF TIME

# FIG. 18

LIGHT DETECTION
INTENSITY Ir

ESTIMATED FINAL LIGHT INTENSITY
(ESTIMATED BIAS QUANTITY)

$C_1$ $C_2$ $C_3$

$I_{rB1}$ $I_{rB2}$ $I_{rB3}$

$I_{rBi}$ : LIGHT RECEPTION INTENSITY AT TIME $t_i$

$C_i$ : HEMOGLOBIN CONCENTRATION AT TIME $t_i$

TIME t

APPLICATION OF PRESSURE

# FIG. 19

CHANGE OF MEASUREMENT DATA WITH PASSAGE OF TIME

# FIG. 20

CHANGE OF HEMOGLOBIN CONCENTRATION
WITH PASSAGE OF TIME

# FIG. 21

SUPERIMPOSITION OF HEMOGLOBIN CONCENTRATION

# FIG. 22

CHANGE OF BLOOD VISCOSITY AND HEMOGLOBIN CONCENTRATION
WITH PASSAGE OF TIME

# FIG. 23

**European Patent Office**

**EUROPEAN SEARCH REPORT**

Application Number

EP 05 10 7015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 178 342 B1 (BORGOS JOHN A ET AL) 23 January 2001 (2001-01-23) * column 1, lines 45-54 * * column 2, lines 25-36 * ----- | 1,2,10, 11 | A61B5/026 |
| P,X | WO 2004/073514 A (HUNTLEIGH TECHNOLOGY PLC; GOUGH, NIGEL) 2 September 2004 (2004-09-02) * page 4, line 5 - page 7, line 6 * ----- | 1,2,10, 11 | |

TECHNICAL FIELDS
SEARCHED      (IPC)

A61B

The present search report has been drawn up for all claims

| Place of search | Date of completion of the search | Examiner |
|---|---|---|
| Munich | 8 November 2005 | Martelli, L |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons

& : member of the same patent family, corresponding
      document

EPO FORM 1503 03.82 (P04C01)

## ANNEX TO THE EUROPEAN SEARCH REPORT
## ON EUROPEAN PATENT APPLICATION NO.

EP 05 10 7015

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2005

| Patent document cited in search report | Publication date | Patent family member(s) | Publication date |
|---|---|---|---|
| US 6178342 B1 | 23-01-2001 | NONE | |
| WO 2004073514 A | 02-09-2004 | AU 2004212761 A1 | 02-09-2004 |